# EUROPEAN PATENT SPECIFICATION

(11) **EP 2 989 213 B1**
(45) Date of publication and mention of the grant of the patent: **06.11.2019**
(21) Application number: 14787882.1
(22) Date of filing: 28.04.2014
(51) Int. Cl.: C12Q 1/6848, C12Q 1/686, C12Q 1/6876

(54) **A METHOD FOR BLOCKING POLYMERASE EXTENSION OF 3 PRIME DNA ENDS BY STEM-LOOP STRUCTURE**
VERFAHREN ZUR BLOCKIERUNG DER POLYMERASEVERLÄNGERUNG VON 3 PRIME-DNA-ENDEN DURCH EINE STAMMSCHLEIFENSTRUKTUR
PROCÉDÉ DE BLOCAGE D'EXTENSION DE POLYMÉRASE D'EXTRÉMITÉS D'ADN AMORCE PAR UNE STRUCTURE TIGE-BOUCLE

(30) Priority: 26.04.2013 US 201361816431 P
(43) Date of publication of application: 02.03.2016
(73) Proprietor: Bio-Rad Laboratories, Inc., Hercules, CA 94547 (US)
(72) Inventor: RAZ, Tal, Brookline, MA 02446 (US); MARY, Pascaline, Cambridge, MA 02139 (US)
(74) Representative: dompatent von Kreisler Selting Werner - Partnerschaft von Patent- und Rechtsanwälten mbB
(86) International application number: PCT/US2014/035730
(87) International publication number: WO 2014/176599

(56) References cited:
- WO-A2-2014/194131
- US-A1- 2012 058 515
- US-A1- 2012 196 279
- US-A1- 2013 040 344
- DAVID E. BIRCH ET AL: "Simplified hot start PCR", NATURE, vol. 381, no. 6581, 30 May 1996 (1996-05-30), pages 445-446, XP055314892, United Kingdom ISSN: 0028-0836, DOI: 10.1038/381445a0
- CHOU Q ET AL: "PREVENTION OF PRE-PCR MIS-PRIMING AND PRIMER DIMERIZATION IMPROVES LOW-COPY-NUMBER AMPLIFICATIONS", NUCLEIC ACIDS RESEARCH, INFORMATION RETRIEVAL LTD, GB, vol. 20, no. 7, 1 January 1992 (1992-01-01), pages 1717-1723, XP002190821, ISSN: 0305-1048
- DON R H ET AL: "'TOUCHDOWN?' PCR TO CIRCUMVENT SPURIOUS PRIMING DURUNG GENE AMPLIFICATION", NUCLEIC ACIDS RESEARCH, INFORMATION RETRIEVAL LTD, GB, vol. 19, no. 14, 1 January 1991 (1991-01-01), page 4008, XP002003542, ISSN: 0305-1048
- HECKER K H ET AL: "HIGH AND LOW ANNEALING TEMPERATURES INCREASE BOTH SPECIFICITY AND YIELD IN TOUCHDOWN AND STEPDOWN PCR", BIOTECHNIQUES RAPID DISPATCHES, INFORMA HEALTHCARE, US, vol. 20, no. 3, 1 March 1996 (1996-03-01), pages 478-485, XP009076187, ISSN: 0736-6205
- LAO K ET AL: "Multiplexing RT-PCR for the detection of multiple miRNA species in small samples", BIOCHEMICAL AND BIOPHYSICAL RESEARCH COMMUNICATIONS, ELSEVIER, AMSTERDAM, NL, vol. 343, no. 1, 28 April 2006 (2006-04-28), pages 85-89, XP024923539, ISSN: 0006-291X, DOI: 10.1016/J.BBRC.2006.02.106 [retrieved on 2006-04-28]

## Description

### BACKGROUND OF THE INVENTION

Nucleic acid assays often utilize DNA polymerase, optionally together with specific DNA probes, to generate an assay signal. For example, an assay may utilize a labelled DNA oligonucleotide as a primer for DNA polymerase. In such assays, the binding and extension of the DNA primer plays a role in assessment of assay results. An example of a potential failure mode in primer-dependent polymerase assays is the priming of DNA polymerase extension in the absence of primer hybridization. This failure can occur as a result of self priming of the DNA target molecule or amplicon or priming by other template molecules in the mix. K. Lao et al., BBRC. 2006, 343:85-89 discloses multiplexing RT-PCR using a stem-loop structure introduced in the template nucleic acid to avoid spurious priming

### BRIEF SUMMARY OF THE INVENTION

Provided herein are methods and compositions for preventing the 3' ends of polynucleic acids from priming polymerase dependent reactions. The method employs polynucleotides having a structure at the 3' end to prevent extension by polymerases.

In some embodiments, a method of performing a primer extension with reduced spurious priming by a primer extension product are provided. In some embodiments, the method comprises:
providing a reaction mixture comprising:
a single-stranded polynucleotide template comprising a 5' and a 3' end, said polynucleotide template comprising, within 6 nucleotides of the 3' end, a double stranded stem of between 5-25 nucleotides, wherein
the double stranded stem comprises a 3' terminus and linked to the 3' terminus of the stem is 1-5 nucleotides that do not form part of the stem and that are at the 3' end of the polynucleotide template, or
a first strand of the double stranded stem is formed by a nucleotide sequence in the polynucleotide template and a second strand of the stem is a separate antisense oligonucleotide complementary to the nucleotide sequence and the antisense oligonucleotide polymerase and wherein the antisense oligonucleotide is complementary up to the 3' end of the polynucleotide template;
a primer that anneals to a target sequence, if present, in the polynucleotide template; and
a polymerase;
exposing the reaction mixture to conditions such that the primer anneals to the target sequence, if present and the polymerase extends the primer in a template-dependent manner if the primer anneals; and
detecting the presence or amount of primer extension product.

The target sequence may be present in the polynucleotide template.

In some embodiments, the double stranded stem is part of a stem loop such that both strands of the double stranded stem are linked by a loop. The loop may comprise 1-10 nucleotides. Further, the double stranded stem may comprise a 3' terminus and the 3' terminus of the stem may be contiguous with the 3' end of the polynucleotide template. In some embodiments, the double stranded stem comprises a 3' terminus and linked to the 3' terminus of the stem is 1-5 nucleotides that do not form part of the stem and that are at the 3' end of the polynucleotide template.

In some embodiments, a first strand of the double stranded stem is formed by a nucleotide sequence in the polynucleotide template and a second strand of the stem is a separate antisense oligonucleotide complementary to the nucleotide sequence and the antisense oligonucleotide is blocked from being extended by a polymerase (e.g., the oligonucleotide does not comprise a 3' -OH moiety). In some embodiments, the double stranded stem comprises a 3' terminus and the 3' terminus of the stem is contiguous with the 3' end of the polynucleotide template.

In some embodiments, the polynucleotide template comprises one member of a quencher/fluorescent label pair and an inhibitor oligonucleotide is annealed to the polynucleotide template downstream of the primer, wherein the inhibitor oligonucleotide comprises a second member of the quencher/fluorescent label pair such that signal from the label is quenched when the inhibitor oligonucleotide is annealed to the polynucleotide template. In some embodiments, the exposing comprises extending the primer with the polymerase, thereby displacing the inhibitor oligonucleotide and unquenching the quencher/fluorescent label pair to generate a fluorescent signal; and detecting the fluorescent signal and correlating the amount of signal to the presence or quantity of the target sequence.

In some embodiments, the polymerase is a DNA polymerase. In some embodiments, the polymerase is an RNA polymerase. In some embodiments, nucleotide triphosphates in the reaction mixture are incorporated into the primer extension product and at least some of said nucleotide triphosphates comprise a label. In some embodiments, nucleotide triphosphates comprise a fluorescent label and quencher pair such that the fluorescent label is quenched by the quencher when the nucleotide triphosphates are intact and the fluorescent label is unquenched when the nucleotide is incorporated into the primer extension product.

Also provided is a single-stranded polynucleotide comprising a 5' and a 3' end, said polynucleotide comprising within 6 nucleotides of the 3' end a double stranded stem of between 5-25 nucleotides, wherein
the double stranded stem comprises a 3' terminus and linked to the 3' terminus of the stem is 1-5 nucleotides that do not form part of the stem and that are at the 3' end of the polynucleotide template, or
a first strand of the double stranded stem is formed by a nucleotide sequence in the polynucleotide template and a second strand of the stem is a separate antisense oligonucleotide complementary to the nucleotide sequence and the antisense oligonucleotide does not comprise a 3' -OH moiety or is otherwise blocked from being extended by a polymerase, and wherein the antisense oligonucleotide is complementary up to the 3' end of the polynucleotide template.

The double stranded stem may be part of a stem loop such that both strands of the double stranded stem are linked by a loop. The loop may comprise 1-10 nucleotides.

The double stranded stem may comprise a 3' terminus and the 3' terminus of the stem may be contiguous with the 3' end of the polynucleotide template.

In some embodiments, the double stranded stem comprises a 3' terminus and linked to the 3' terminus of the stem is 1-5 nucleotides that do not form part of the stem and that are at the 3' end of the polynucleotide template.

In some embodiments, a first strand of the double stranded stem is formed by a nucleotide sequence in the polynucleotide template and a second strand of the stem is a separate anti-sense oligonucleotide complementary to the nucleotide sequence and the antisense oligonucleotide does not comprise a 3' -OH moiety or is otherwise blocked from being extended by a polymerase.

The double stranded stem may comprise a 3' terminus and the 3' terminus of the stem may be contiguous with the 3' end of the polynucleotide template.

The polynucleotide template may comprise one member of a quencher/fluorescent label pair.

The single-stranded polynucleotide may be between 10-5000 nucleotides long.

Also provided is a reaction mixture comprising the single-stranded polynucleotide as described above. In some embodiments, the polynucleotide comprises one member of a quencher/fluorescent label pair and the reaction mixture further comprises an inhibitor oligonucleotide, wherein the inhibitor oligonucleotide comprises a second member of the quencher/fluorescent label pair such that signal from the label is quenched when the inhibitor oligonucleotide is annealed to the polynucleotide. IThe reaction mixture may further comprise at least one of (or all of): a DNA or RNA polymerase; nucleotide triphosphates or deoxynucleotide triphosphates; a primer that anneals to a target sequence in the single-stranded polynucleotide; or an inhibitor oligonucleotide, wherein the inhibitor oligonucleotide comprises a second member of the quencher/fluorescent label pair such that signal from the label is quenched when the inhibitor oligonucleotide is annealed to the polynucleotide.

Also disclosed are kits comprising the single-stranded polynucleotide as described above.

Also disclosed is a method of making the single-stranded polynucleotide as described above. The method may comprise, contacting a nucleic acid sample comprising the template nucleic acid with a DNA polymerase and at least a first oligonucleotide primer to form a mixture, said first oligonucleotide primer comprising a stem loop, said primer having a 5' end and a 3' end and comprising from the 5' end to the 3' end: optionally 1-6 nucleotides at the 5' end that do not form part of the stem loop, followed by nucleotides forming a first half of the stem of the stem loop, followed by nucleotides forming the loop of the stem loop, followed by nucleotides forming a second half of the stem that hybridizes to the first half of the stem loop, followed by a sequence at the 3' end complementary to the template nucleic acid; and exposing the mixture to conditions such that the 3' end of the first oligonucleotide primer anneals to the template nucleic acid and the DNA polymerase extends the 3' end of the first oligonucleotide primer in a template-dependent manner to form a single-stranded amplicon comprising the first oligonucleotide primer and a complement of the template nucleic acid, thereby amplifying the template nucleic acid. The mixture may further comprise a second oligonucleotide primer comprising a sequence complementary to the single-stranded amplicon and wherein the exposing results in annealing the second oligonucleotide primer to the single-stranded amplicon and extending the second oligonucleotide primer with the DNA polymerase to form a polynucleotide that is complementary to the single-stranded amplicon and having a stem loop at the 5' end of the polynucleotide, thereby forming a double-stranded amplicon.

The second oligonucleotide primer may have a 3' and a 5' end, and the second oligonucleotide primer may comprise a stem loop ending within three nucleotides of the 5' end of the second oligonucleotide primer.

The exposing may comprise the polymerase chain reaction (PCR).

The first or second oligonucleotide may comprise a label such that at least one strand of the double-stranded amplicon comprises the label. The label may be fluorescent.

The first or second oligonucleotide primer may comprise a quencher such that at least one strand of the double-stranded amplicon comprises the quencher.

The first half and the second half of the stem may each have 5-15 nucleotides. Between 2-6 nucleotides may form the loop of the stem loop. In some cases, only one nucleotide at the 5' end of the first oligonucleotide primer may not form part of the stem loop. In some cases, two nucleotides at the 5' end of the first oligonucleotide primer may not form part of the stem loop.

The sequence at the 3' end of the first oligonucleotide primer and complementary to the template nucleic acid may be at least 6 nucleotides long.

Also disclosed is an oligonucleotide primer comprising a stem loop, said primer having a 5' end and a 3' end and comprising from the 5' end to the 3' end: optionally 1-6 nucleotides at the 5' end that do not form part of the stem loop, followed by a first half of the stem of the stem loop, followed by nucleotides forming the loop of the stem loop, followed by a second half of the stem being the reverse complement of the first half of the stem loop, followed by a sequence at the 3' end (e.g., complementary to a template nucleic acid).

The first half and the second half of the stem may each have 5-15 nucleotides. In some cases, between 2-6 nucleotides may form the loop of the stem loop. In some cases, only one nucleotide at the 5' end of the first oligonucleotide primer may not form part of the stem loop. In some cases, two nucleotides at the 5' end of the first oligonucleotide primer may not form part of the stem loop. In some cases, the sequence at the 3' end of the first oligonucleotide primer and complementary to the template nucleic acid may be at least 6 nucleotides long.

Also disclosed is a reaction mixture comprising the oligonucleotide primer as described. The reaction mixture mayfurther comprises one or more of: a DNA polymerase; or a second oligonucleotide having a sequence different from said oligonucleotide primer.

Also disclosed is kit a comprising the oligonucleotide primer as described above.

### DEFINITIONS

Unless defined otherwise, all technical and scientific terms used herein generally have the same meaning as commonly understood by one of ordinary skill in the art to which this invention belongs. Generally, the nomenclature used herein and the laboratory procedures in cell culture, molecular genetics, organic chemistry, and nucleic acid chemistry and hybridization described below are those well known and commonly employed in the art. Standard techniques are used for nucleic acid and peptide synthesis. The techniques and procedures are generally performed according to conventional methods in the art and various general references (see generally, Sambrook et al. MOLECULAR CLONING: A LABORATORY MANUAL, 2d ed. (1989) Cold Spring Harbor Laboratory Press, Cold Spring Harbor, N.Y.), which are provided throughout this document. The nomenclature used herein and the laboratory procedures in analytical chemistry, and organic synthetic described below are those well known and commonly employed in the art.

The term "amplification reaction" refers to any in vitro means for multiplying the copies of a target sequence of nucleic acid. Such methods include polymerase chain reaction (PCR), DNA ligase chain reaction (see U.S. Patents 4,683,195 and 4,683,202; PCR Protocols: A Guide to Methods and Applications (Innis et al., eds, 1990)), (LCR), QBeta RNA replicase, and RNA transcription-based (such as TAS and 3SR) amplification reactions as well as others known to those of skill in the art.

"Amplifying" refers to a step of submitting a solution to conditions sufficient to allow for amplification of a polynucleotide if all of the components of the reaction are intact. Components of an amplification reaction include, e.g., primers, a polynucleotide template, polymerase and nucleotides. The term "amplifying" typically refers to an "exponential" increase in target nucleic acid. However, "amplifying" as used herein can also refer to linear increases in the numbers of a select target sequence of nucleic acid, such as is obtained with cycle sequencing.

The term "amplification reaction mixture" refers to an aqueous solution comprising the various reagents used to amplify a target nucleic acid. These include enzymes, aqueous buffers, salts, amplification primers, target nucleic acid, and nucleoside triphosphates. Amplification reaction mixtures may also further include stabilizers and other additives to optimize efficiency and specificity. Depending upon the context, the mixture can be either a complete or incomplete amplification reaction mixture

"Polymerase chain reaction" or "PCR" refers to a method whereby a specific segment or subsequence of a target double-stranded DNA, is amplified in a geometric progression. PCR is well known to those of skill in the art; see, e.g., U.S. Patents 4,683,195 and 4,683,202; and PCR Protocols: A Guide to Methods and Applications, Innis et al., eds, 1990. Exemplary PCR reaction conditions typically comprise either two or three step cycles. Two step cycles have a denaturation step followed by a hybridization/elongation step. Three step cycles comprise a denaturation step followed by a hybridization step followed by a separate elongation step.

A "primer" refers to a polynucleotide sequence that hybridizes to a sequence on a target nucleic acid and serves as a point of initiation of nucleic acid synthesis. Primers can be of a variety of lengths and are often less than 50 nucleotides in length, for example 12-30 nucleotides, in length. The length and sequences of primers for use in PCR can be designed based on principles known to those of skill in the art, see, e.g., Innis et al., supra.

A "template" refers to a polynucleotide sequence that comprises the polynucleotide to be amplified, flanked by or a pair of primer hybridization sites. Thus, a "target template" comprises the target polynucleotide sequence flanked by hybridization sites for a "forward" primer and a "reverse" primer.

As used herein, "nucleic acid" means DNA, RNA, single-stranded, double-stranded, or more highly aggregated hybridization motifs, and any chemical modifications thereof. Modifications include those providing chemical groups that incorporate additional charge, polarizability, hydrogen bonding, electrostatic interaction, points of attachment and functionality to the nucleic acid ligand bases or to the nucleic acid ligand as a whole. Such modifications include peptide nucleic acids (PNAs), phosphodiester group modifications (e.g., phosphorothioates, methylphosphonates), 2'-position sugar modifications, 5-position pyrimidine modifications, 8-position purine modifications, modifications at exocyclic amines, substitution of 4-thiouridine, substitution of 5-bromo or 5-iodo-uracil; backbone modifications, methylations, unusual base-pairing combinations such as the isobases, isocytidine and isoguanidine. Nucleic acids can also include non-natural bases, such as, for example, nitroindole. Modifications can also include 3' and 5' modifications including capping with a fluorophore (e.g., quantum dot) or another moiety.

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymers.

A "polymerase" refers to an enzyme that performs template-directed synthesis of polynucleotides, e.g., DNA and/or RNA. The term encompasses both the full length polypeptide and a domain that has polymerase activity. DNA polymerases are well-known to those skilled in the art, including DNA polymerases isolated or derived from *Pyrococcus furiosus*, *Thermococcus litoralis*, and *Thermotoga maritime*. Additional examples of commercially available polymerase enzymes include: Klenow fragment (New England Biolabs® Inc.), Taq DNA polymerase (QIAGEN), 9°N™ DNA polymerase (New England Biolabs® Inc.), Deep Vent™ DNA polymerase (New England Biolabs® Inc.), Manta DNA polymerase (Enzymatics®), Bst DNA polymerase (New England Biolabs® Inc.), and phi29 DNA polymerase (New England Biolabs® Inc.). Polymerases include both DNA-dependent polymerases and RNA-dependent polymerases such as reverse transcriptase. At least five families of DNA-dependent DNA polymerases are known, although most fall into families A, B and C. There is little or no sequence similarity among the various families. Most family A polymerases are single chain proteins that can contain multiple enzymatic functions including polymerase, 3' to 5' exonuclease activity and 5' to 3' exonuclease activity. Family B polymerases typically have a single catalytic domain with polymerase and 3' to 5' exonuclease activity, as well as accessory factors. Family C polymerases are typically multi-subunit proteins with polymerizing and 3' to 5' exonuclease activity. In E. coli, three types of DNA polymerases have been found, DNA polymerases I (family A), II (family B), and III (family C). In eukaryotic cells, three different family B polymerases, DNA polymerases α, δ, and ε, are implicated in nuclear replication, and a family A polymerase, polymerase γ, is used for mitochondrial DNA replication. Other types of DNA polymerases include phage polymerases. Similarly, RNA polymerases typically include eukaryotic RNA polymerases I, II, and III, and bacterial RNA polymerases as well as phage and viral polymerases. RNA polymerases can be DNA-dependent and RNA-dependent.

The terms "label," "detectable label, " and "detectable moiety" refer to a composition detectable by spectroscopic, photochemical, biochemical, immunochemical, chemical, or other physical means. For example, useful labels include fluorescent dyes (fluorophores), luminescent agents, electron-dense reagents, enzymes (e.g., as commonly used in an ELISA), biotin, digoxigenin, ³²P and other isotopes, haptens, and proteins which can be made detectable, *e*.*g.*, by incorporating a radio label into the peptide or used to detect antibodies specifically reactive with the peptide. The term includes combinations of single labeling agents, *e.g.,* a combination of fluorophores that provides a unique detectable signature, *e*.*g*., at a particular wavelength or combination of wavelengths. Any method known in the art for conjugating label to a desired agent may be employed, *e*.*g*., using methods described in Hermanson, Bioconjugate Techniques 1996, Academic Press, Inc., San Diego.

A "stem loop," also known as a "hairpin" or "hairpin loop," refers to a secondary structure formed by a single-stranded oligonucleotide when complementary bases in a first part of the linear strand hybridize with bases in a second part of the same strand. The sequence in the second part of the sequences is the reverse complement of the first part sequence, thereby allowing for hybridization.

As used herein, the term "partitioning" or "partitioned" refers to separating a sample into a plurality of portions, or "partitions." Partitions can be solid or fluid. A partition may be a solid partition, *e.g*., a microchannel. A partition may be a fluid partition, *e.g*., a droplet. A fluid partition (*e*.*g.*, a droplet) may be a mixture of immiscible fluids (*e.g*., water and oil). A fluid partition (*e*.*g*., a droplet) may be an aqueous droplet that is surrounded by an immiscible carrier fluid (*e*.*g.*, oil).

### BRIEF DESCRIPTION OF THE DRAWINGS

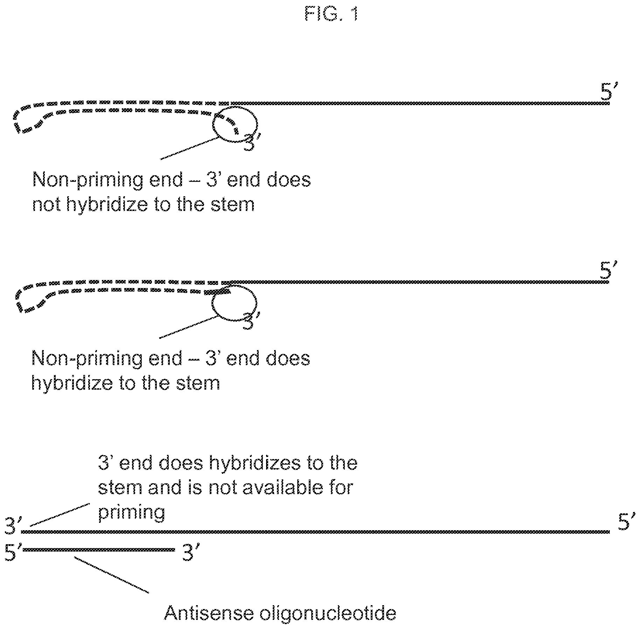

FIG. 1 illustrates three possibilities for blocking spurious priming from a polynucleotide. In the top of the figure, the polynucleotide has a stem loop structure, with the last few nucleotides of the 3' end not forming the stem. In the middle of the figure, the polynucleotide has a stem loop structure, with the nucleotide of the 3' end forming the last portion of the stem (the 3' end of the polynucleotide and the 3 terminus of the stem are contiguous). In the bottom of the figure, a double stranded stem occurs at the 3' end of the polynucleotide but a loop does not link the two strands.

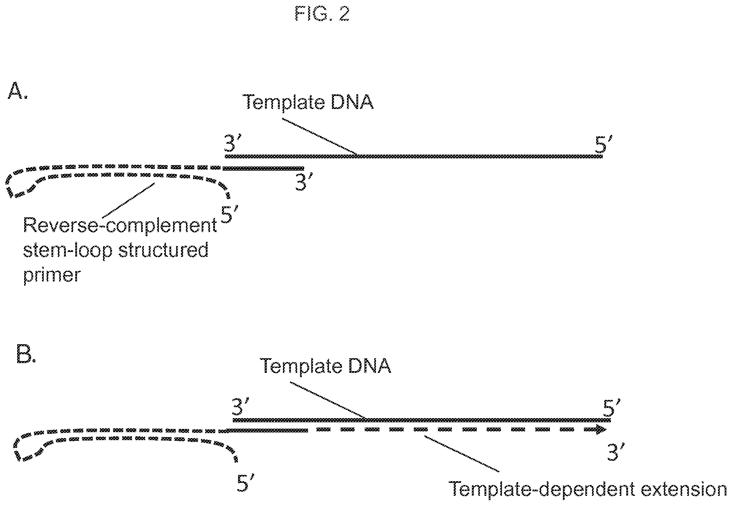

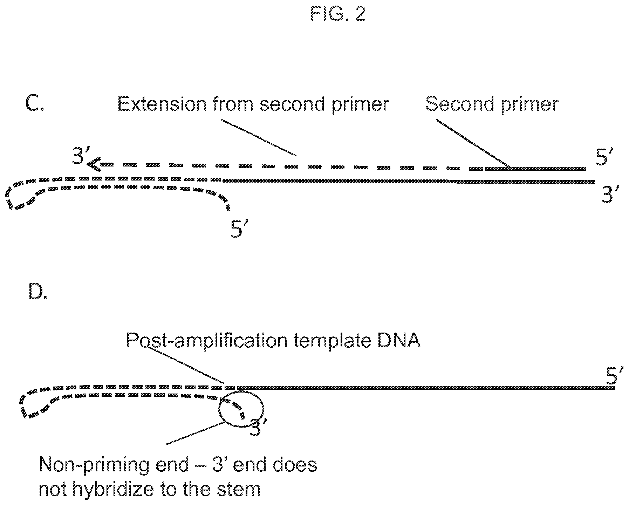

FIG. 2 illustrates incorporation of the 3' stem-loop DNA structure into an amplicon as one of several methods to add the 3' stem structure. The stem-loop structure is introduced with the PCR primer as shown in part A. The 5' end of the primer is optionally designed so that it will not hybridize to the stem structure. Part B of FIG. 2 illustrates template-dependent extension of the stem loop primer to form one strand of the amplicon. FIG. 2, part C illustrates extension of a second primer, which is complementary to the single-stranded amplicon. This extension continues along the original primer sequence. FIG. 2 part D illustrates the resulting polynucleotide that is complementary to the single-stranded amplicon. This resulting sequence contains a stem loop-blocked 3' end. Note that in cases (not illustrated in the figure) where the second primer also contains a 5' stem loop structure, both strands of the resulting amplicon would have stem loop-blocked 3' ends.

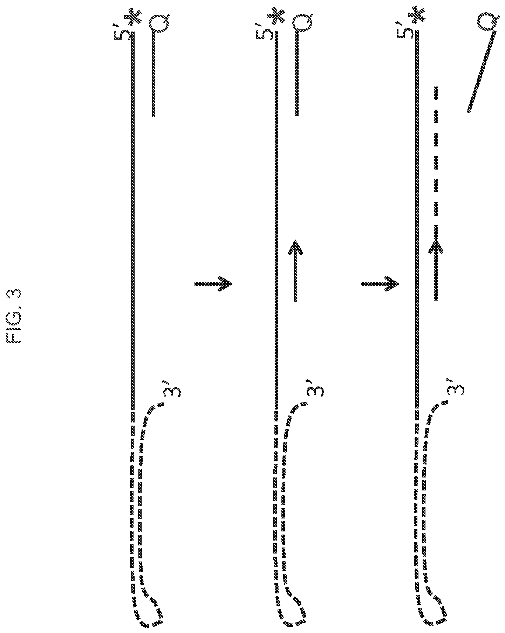

FIG. 3 illustrates an exemplary strand displacement assay using a template polynucleotide having a 3' stem loop near the 3' terminus. As discussed elsewhere herein, the stem loop can be present at the very 3' end or there can be a few nucleotides (e.g., 1-6) at the 3' end that are not part of the stem. In yet other cases, the template can have a double-stranded stem without the loop linking the two strands. In the top portion of the figure, the template polynucleotide is fluorescently labeled (*) at the 5' end and an inhibitor oligonucleotide comprising a quencher (Q) is annealed. In the middle of the figure, a primer is annealed to a target sequence, if present, in the template polynucleotide. In the bottom of the figure, the primer is extended with a polymerase, thereby displacing the inhibitor molecule and unquenching the fluorescent label (*).

### DETAILED DESCRIPTION OF THE INVENTION

The presence of polymerase-extendable 3' ends in DNA templates can increase the background noise in assays that rely on DNA-polymerase extension. The methods and compositions described herein provide a non-priming stable stem loop DNA structure or a double-stranded stem structure at the 3' end of amplified template DNA, thereby reducing or eliminating spurious priming from the 3' end. Template polynucleotides comprising the stem or stem loop structure at the 3' end can then be used in primer extension reactions in which a polymerase extends the primer annealed to template polynucleotide without spurious priming events caused by the 3' end of the template.

Also provided are various methods of advantageously using the template polynucleotides in improved primer extension methods as well as reaction mixtures for their use.

### 3' stem-loop polynucleotides

As noted above, in some embodiments, a template polynucleotide is provided having a stem loop at or near the 3' end of the polynucleotide. The inventors have tested several variants of such stem loop polynucleotides and have found that the stem loop can be contiguous with the 3' end of the polynucleotide or there can be a few nucleotides at the 3' end that are not part of the stem, i.e., are after the stem loop. In both cases, spurious 3' priming from the 3' end of the polynucleotide is reduced, with a greater reduction observed wherein there are a few nucleotides at the 3' end that are not part of the stem.

Accordingly, the polynucleotide can be composed of several sections:
The stem loop portion will comprise a sequence forming the first half of the stem, followed by a number of nucleotides forming the loop followed by a reverse complement of the first half of the stem. The first half and second half of the stem will generally be the exact same length though, one half can have one nucleotide more than the other stem half such that when the two halves anneal, one nucleotide of one half does not anneal and forms a bulge. Each half of the stem can be any size as desired, for example, between 5-15 nucleotides long, e.g., 5, 6, 7, 8, 9, 10, 11, 12, 13, 14, or 15 nucleotides. The loop will have at least one nucleotide, and in some cases, between 2-6, e.g., 2, 3, 4, 5, or 6 nucleotides. In some cases, the stem will have a melting temperature (Tm) of at least 40, 45, 50, 55, 60, or 65°C. For example, the Tm may be at least 5 or 10 degrees higher than the temperature of the primer extension assay employed. The Tm of the loop can be determined empirically, and can be estimated, e.g., using formulas as described in F. Baldino, Jr, M.-F. Chesselet, and M.E. Lewis, Methods in Enzymology 168:766 (1989). The stem may have a minimum free energy (delta-G) of -8.5, -3, or less at 37 °C. Ideally, the polynucleotide 3' end sequence will allow for few or no alternative conformations (e.g., other alternative secondary structures). These aspects can be analyzed by software available to those of ordinary skill in the art. Examples of such software include the UNAFold software available at mfold.ma.albany.edu. Alternatively, more complicated (e.g., hammerhead) secondary structures can be formed so long as the structure impairs availability of the 3' end to reduce spurious priming.
The final 3' end of the polynucleotide may coincide with the 3' terminus of the stem (i.e., the last nucleotide in the stem structure). Alternatively, at the 3' end, 1, 2, 3, 4, 5 or 6 nucleotides can be present at the 5' end that is/are not part of the stem loop due to lack of complementarity to nearby nucleotides in the 5' direction of the stem. This aspect is illustrated, for example in the top of FIG. 1.

The length of the polynucleotide comprising the stem loop or stem at the 3' end can vary as needed. The polynucleotide may be between 10-5000, 30-5000, 30-100, 50-1000, 50-500, or 50-1000 nucleotides long.

### 3' stem (no loop) polynucleotides

In other embodiments, an antisense-oligonucleotide complementary to the 3' portion, and up to the 3' end, of the polynucleotide can be annealed to the polypeptide. This aspect is depicted, for example, in FIG. 1, bottom portion. The antisense oligonucleotide and complementary portion of the polynucleotide form a duplex that is analogous to the stem of a stem loop, but without a loop of nucleotides linking the two nucleic acids. Each half of the stem can be any size as desired, for example, between 5-30 nucleotides long, e.g., 5, 6, 7, 8, 9, 10, 11, 12, 13, 14, 15, 16, 17, 18, 19, 20, 21, 22, 23, 24, 25, 26, 27, 28, 29, or 30 nucleotides. The stem may have a melting temperature (Tm) of at least 40, 45, 50, 55, 60, or 65°C. For example, the Tm may be at least 5 or 10 degrees higher than the temperature of the primer extension assay employed. The stem may have a minimum free energy (delta-G) of -8.5 or less at 37° C. Ideally, the polynucleotide 3' end sequence will allow for few or no alternative conformations (e.g., other alternative secondary structures). Alternatively, more complicated (e.g., hammerhead) secondary structures can be formed so long as the structure impairs availability of the 3' end to reduce spurious priming.

### Methods of using the 3' stem-loop or stem polynucleotides

The polynucleotides described above have reduced ability to prime polymerase extension from their 3' end, and thus are desirably used in any method for which priming by a template polynucleotide interferes with signal of a reaction. As shown in the Example below, the template polynucleotides having an incorporated 3' stem loop structure allow for more accurate and consistent assays that subsequently use a polymerase and that are susceptible to interference from the template 3' end.

Accordingly, methods of using the resulting polynucleotides are also provided. For example, in some embodiments, the polynucleotide having the incorporated 3' stem loop or stem structure is used in an inhibitor displacement reaction. An example of such a method is described in PCT Publication WO2012/078710. Briefly, these methods can involve a template polynucleotide having a 3' stem or stem loop structure as described above and comprising one of a quencher/fluorescent label pair. For example the template polynucleotide can have a fluorescent label, or the template polynucleotide can have a quencher. An inhibitor polynucleotide comprising the other part of the quencher/fluorescent label pair is hybridized to the template, resulting in quenching of the fluorescent label signal by the nearby quencher. A primer targeted upstream of the annealed inhibitor polynucleotide can be used in combination with a polymerase to detect the presence or absence of a particular target sequence within the template polynucleotide. If the primer hybridizes to the template, the resulting polymerase reaction displaces the inhibitor polynucleotide, resulting in signal from the fluorescent label. The primer may hybridize to a location of the single nucleotide polymorphism (SNP) under conditions in which the primer anneals if the primer is complementary to a particular SNP allele and does not anneal to an alternative SNP allele. An aspect of this method is depicted in FIG. 3. Surprisingly, as shown in the Example below, in some cases, the template polynucleotide 3' end can self-prime the reaction, resulting in displacement of the inhibitor polynucleotide in the absence of an upstream primer. This undesirable self-priming activity can be significantly reduced by incorporating a 3' stem loop structure into the template polynucleotide before performing the inhibitor polynucleotide displacement assay.

In another embodiment, the polynucleotide having the incorporated 3' stem loop or stem structure is used as a template for a primer extension reaction in which the nucleotides are labeled. For example, in some embodiments, nucleotide triphosphates are linked to a fluorescent label and a quencher such that signal from the label is quenched. Incorporation of the nucleotide triphosphate into an extension product comprises incorporating a nucleotide monophosphate and release of a diphosphate. In aspects in which the quencher is linked to the diphosphate portion of the nucleotide triphosphate, incorporation of the nucleotide into the extension product results in release of the quencher thereby generating signal from the fluorescent label. Examples of nucleotides having a fluorescent label and quencher are described in, e.g., US Patent 7,118,871 and D.A. Berry, et al., Tetrahedron Lett, 45:2457-2461 (2004). Fluorescence can be detected and quantified as desired and optionally correlated with the quantity of original template or primer-binding target sequence on the template. Depending on the template and labeled nucleotides employed, a DNA or RNA polymerase can be used.

The methods described herein can be used to detect nucleic acid in any type of sample. The sample may be a biological sample. Biological samples can be obtained from any biological organism, *e*.*g*., an animal, plant, fungus, bacterial, or any other organism. The biological sample may be from an animal, *e.g*., a mammal (*e*.*g*., a human or a non-human primate, a cow, horse, pig, sheep, cat, dog, mouse, or rat), a bird (*e*.*g*., chicken), or a fish. The nucleic acid template can be RNA or DNA.

A biological sample can be any tissue or bodily fluid obtained from a biological organism, *e.g.,* blood, a blood fraction, or a blood product (*e*.*g*., serum, plasma, platelets and red blood cells), sputum or saliva, tissue (*e*.*g*., kidney, lung, liver, heart, brain, nervous tissue, thyroid, eye, skeletal muscle, cartilage, or bone tissue), cultured cells, stool and urine. The sample may comprise one or more cells. The cells may be animal cells, including human, or non-human, mammalian cells. Non-human mammalian cells include primate cells, mouse cells, rat cells, porcine cells, and bovine cells. The cells may be plant or fungal (including yeast) cells. Cells can be, for example, cultured primary cells, immortalized culture cells, or cells from a biopsy or tissue sample, optionally cultured and stimulated to divide before assayed.

The template may be amplified under ambient conditions (e.g., below 45 °C) using a temperature sensitive DNA polymerase. The primer extension reaction may comprise amplifying the template nucleic acid under isothermal or thermocyclic conditions. Amplifying the nucleic acid molecules or regions of the nucleic acid molecule may comprise polymerase chain reaction (PCR), quantitative PCR, or real-time PCR. Protocols for carrying out PCR are described, for example, in PCR Primer: A Laboratory Manual, Dieffenbach and Dveksler, eds., 2003, Cold Spring Harbor Laboratory Press; A-Z of Quantitative PCR, Bustin, ed., 2004, International University Line; Edwards, Real-Time PCR: An Essential Guide, 2004, Taylor & Francis; Real Time PCR, Dorak, ed., 2006, Taylor & Francis; PCR Protocols: A Guide to Methods and Applications, Innis, et al., eds., 1990, Academic Press, San Diego; PCR Strategies, Innis, et al., eds, 1995, Academic Press, San Diego; and PCR Applications: Protocols for Functional Genomics, Innis, et al., eds., 1999, Academic Press, San Diego.

Quantitative amplification (including real-time PCR) methods are used for determination of the amount of nucleic acid molecules or regions of a nucleic acid molecule that co-localize in a compartment, and can be used with various controls to determine the relative amount of co-localization of nucleic acid molecules or regions of a nucleic acid molecule in a sample of interest, thereby indicating whether and to what extent nucleic acids in a sample are in close proximity to each other.

Quantitative amplification methods (e.g., quantitative PCR or quantitative linear amplification) involve amplification of nucleic acid template, directly or indirectly (e.g., determining a Ct value) determining the amount of amplified DNA, and then calculating the amount of initial template based on the number of cycles of the amplification. Amplification of a DNA locus using reactions is well known (see U.S. Patents 4,683,195 and 4,683,202; PCR PROTOCOLS: A GUIDE TO METHODS AND APPLICATIONS (Innis et al., eds, 1990)). Typically, PCR is used to amplify DNA templates. However, alternative methods of amplification have been described and can also be employed. Methods of quantitative amplification are disclosed in, e.g., U.S. Patents 6,180,349; 6,033,854; and 5,972,602, as well as in, e.g., Gibson et al., Genome Research 6:995-1001 (1996); DeGraves, et al., Biotechniques 34(1):106-10, 112-5 (2003); Deiman B, et al., Mol Biotechnol. 20(2):163-79 (2002). Amplifications can be monitored in "real time."

The sample or mixture comprising the template polynucleotide may be partitioned into a plurality of partitions. Partitioning can be used, for example, to limit the number of copies of a template polynucleotide or other limiting reagent per partition. Partitions can include any of a number of types of partitions, including solid partitions (*e.g*., wells or tubes) and fluid partitions (*e.g*., aqueous droplets within an oil phase). the partitions may be droplets. The partitions may be microchannels. Methods and compositions for partitioning a sample are described, for example, in published patent applications WO 2010/036352, US 2010/0173394, US 2011/0092373, and US 2011/0092376.

The sample may be partitioned into a plurality of droplets. A droplet may comprise an emulsion composition, *i.e*., a mixture of immiscible fluids (*e.g*., water and oil). A droplet may be an aqueous droplet that is surrounded by an immiscible carrier fluid (*e*.*g*., oil). A droplet may be an oil droplet that is surrounded by an immiscible carrier fluid (*e*.*g*., an aqueous solution). The droplets described herein may be relatively stable and have minimal coalescence between two or more droplets. Less than 0.0001 %, 0.0005 %, 0.001 %, 0.005 %,0.01 %,0.05 %, 0.1 %,0.5 %, 1%, 2%, 3%, 4%, 5%, 6%, 7%, 8%, 9 %, or 10 % of droplets generated from a sample coalesce with other droplets. The emulsions can also have limited flocculation, a process by which the dispersed phase comes out of suspension in flakes.

The droplet may be formed by flowing an oil phase through an aqueous sample comprising the target molecule(s) to be detected. The aqueous sample comprising the target molecule(s) to be detected may further comprise a buffered solution and two or more probes for detecting the target molecule(s).

The oil phase may comprise a fluorinated base oil which may additionally be stabilized by combination with a fluorinated surfactant such as a perfluorinated polyether. The base oil may comprise one or more of a HFE 7500, FC-40, FC-43, FC-70, or another common fluorinated oil. The oil phase may comprise an anionic fluorosurfactant. The anionic fluorosurfactant may be Ammonium Krytox (Krytox-AS), the ammonium salt of Krytox FSH, or a morpholino derivative of Krytox FSH. Krytox-AS may be present at a concentration of about 0.1%, 0.2%, 0.3%, 0.4%, 0.5%, 0.6%, 0.7%, 0.8%, 0.9%, 1.0%, 2.0 %, 3.0 %, or 4.0 % (w/w). The concentration of Krytox-AS may be about 1.8 %. The concentration of Krytox-AS may be about 1.62 %. Morpholino derivative of Krytox FSH may be present at a concentration of about 0.1%, 0.2%, 0.3%, 0.4%, 0.5%, 0.6%, 0.7%, 0.8 %, 0.9 %, 1.0 %, 2.0 %, 3.0 %, or 4.0 % (w/w). The concentration of morpholino derivative of Krytox FSH may be about 1.8 %. The concentration of morpholino derivative of Krytox FSH may be about 1.62 %.

The oil phase may further comprise an additive for tuning the oil properties, such as vapor pressure, viscosity, or surface tension. Examples include perfluorooctanol and 1H, 1H,2H,2H-Perfluorodecanol. 1H, 1H,2H,2H-Perfluorodecanol may be added to a concentration of about 0.05 %, 0.06 %, 0.07 %, 0.08 %, 0.09 %, 0.1 %, 0.2 %, 0.3 %, 0.4 %, 0.5%, 0.6%, 0.7 %, 0.8 %, 0.9 %, 1.0 %, 1.25 %, 1.50 %, 1.75%, 2.0%, 2.25%, 2.5%, 2.75 %, or 3.0 % (w/w). 1H,1H,2H,2H-Perfluorodecanol may be added to a concentration of about 0.18 % (w/w).

The emulsion may be formulated to produce highly monodisperse droplets having a liquid-like interfacial film that can be converted by heating into microcapsules having a solid-like interfacial film; such microcapsules may behave as bioreactors able to retain their contents through an incubation period. The conversion to microcapsule form may occur upon heating. For example, such conversion may occur at a temperature of greater than about 40 °C, 50 °C, 60 °C, 70 °C, 80 °C, 90 °C, or 95 °C. During the heating process, a fluid or mineral oil overlay may be used to prevent evaporation. Excess continuous phase oil may or may not be removed prior to heating. The biocompatible capsules may be resistant to coalescence and/or flocculation across a wide range of thermal and mechanical processing.

Following conversion, the microcapsules may be stored at about -70 °C, -20 °C, 0 °C, 3 °C, 4 °C, 5 °C, 6 °C, 7 °C, 8 °C, 9 °C, 10 °C, 15 °C, 20 °C, 25 °C, 30 °C, 35 °C, or 40 °C. These capsules may be useful in biomedical applications, such as stable, digitized encapsulation of macromolecules, particularly aqueous biological fluids comprising a mix of target molecules such as nucleic acids, proteins, or both together; drug and vaccine delivery; biomolecular libraries and clinical imaging applications.

The microcapsule partitions may contain one or more probes or labeled primers and may resist coalescence, particularly at high temperatures. Accordingly, the capsules can be incubated at a very high density (*e*.*g*., number of partitions per unit volume). Greater than 100,000, 500,000, 1,000,000, 1,500,000, 2,000,000, 2,500,000, 5,000,000, or 10,000,000 partitions may be incubated per ml. The sample-probe incubations may occur in a single well, e.g., a well of a microtiter plate, without inter-mixing between partitions. The microcapsules may also contain other components necessary for the incubation.

The sample may be partitioned into at least 500 partitions, at least 1000 partitions, at least 2000 partitions, at least 3000 partitions, at least 4000 partitions, at least 5000 partitions, at least 6000 partitions, at least 7000 partitions, at least 8000 partitions, at least 10,000 partitions, at least 15,000 partitions, at least 20,000 partitions, at least 30,000 partitions, at least 40,000 partitions, at least 50,000 partitions, at least 60,000 partitions, at least 70,000 partitions, at least 80,000 partitions, at least 90,000 partitions, at least 100,000 partitions, at least 200,000 partitions, at least 300,000 partitions, at least 400,000 partitions, at least 500,000 partitions, at least 600,000 partitions, at least 700,000 partitions, at least 800,000 partitions, at least 900,000 partitions, at least 1,000,000 partitions, at least 2,000,000 partitions, at least 3,000,000 partitions, at least 4,000,000 partitions, at least 5,000,000 partitions, at least 10,000,000 partitions, at least 20,000,000 partitions, at least 30,000,000 partitions, at least 40,000,000 partitions, at least 50,000,000 partitions, at least 60,000,000 partitions, at least 70,000,000 partitions, at least 80,000,000 partitions, at least 90,000,000 partitions, at least 100,000,000 partitions, at least 150,000,000 partitions, or at least 200,000,000 partitions.

The sample may be partitioned into a sufficient number of partitions such that primer extension can be distinguished from random co-localization. The partitioning may comprise generating at least 1 partition that has 0 copies of the template molecule. The partitioning may comprise generating at least about 1, 2, 3, 4, 5, 6, 7, 8, 9, 10, 15, 20, 25, 30, 40, 50, 60, 70, 80, 90, 100, 150, 200, 250, 300, 400, 500, 600, 700, 800, 900, or 1000 partitions or more that have 0 copies of the template molecule. The partitioning may comprise generating at least about 1, 2, 3, 4, 5, 6, 7, 8, 9, 10, 15, 20, 25, 30, 40, 50, 60, 70, 80, 90, 100, 150, 200, 250, 300, 400, 500, 600, 700, 800, 900, or 1000 partitions or more that have 0 copies of the template molecule and at least about 1, 2, 3, 4, 5, 6, 7, 8, 9, 10, 15, 20, 25, 30, 40, 50, 60, 70, 80, 90, 100, 150, 200, 250, 300, 400, 500, 600, 700, 800, 900, or 1000 partitions or more that have 1 or more copies (*e*.*g*., 1, 2, 3, 4, 5, 6, 7, 8, 9, 10 or more copies) of the template molecule.

The sample may be partitioned into a sufficient number of partitions such that at least a majority of partitions have no more than 5 copies of the template molecule (e.g., about 0.5, 1, 2, 3, 4, or 5 copies of the target molecule). A majority of the partitions may have no more than 5 copies of the one or more template molecules to be detected. On average no more than 5 copies of the one or more template molecules may be present in each partition. On average about 0.5, 1, 2, 3, 4, or 5 copies of the template molecule may be present in each partition. On average about 0.001, 0.005, 0.01, 0.05, 0.1, 0.5, 1, 2, 3, 4, or 5 probes may be present in each partition.

The droplets that are generated are substantially uniform in shape and/or size. For example, the droplets may be substantially uniform in average diameter. The droplets that are generated may have an average diameter of about 0.001 µm, about 0.005 µm, about 0.01 µm, about 0.05 µm, about 0.1 µm, about 0.5 µm, about 1 µm, about 5 µm, about 10 µm, about 20 µm, about 30 µm, about 40 µm, about 50 µm, about 60 µm, about 70 µm, about 80 µm, about 90 µm, about 100 µm, about 150 µm, about 200 µm, about 300 µm, about 400 µm, about 500 µm, about 600 µm, about 700 µm, about 800 µm, about 900 µm, or about 1000 µm. The droplets that are generated may have an average diameter of less than about 1000 µm, less than about 900 µm, less than about 800 µm, less than about 700 µm, less than about 600 µm, less than about 500 µm, less than about 400 µm, less than about 300 µm, less than about 200 µm, less than about 100 µm, less than about 50 µm, or less than about 25 µm. The droplets that are generated may be non-uniform in shape and/or size.

The droplets that are generated may be substantially uniform in volume. For example, the droplets that are generated may have an average volume of about 0.001 nl, about 0.005 nl, about 0.01 nl, about 0.02 nl, about 0.03 nl, about 0.04 nl, about 0.05 nl, about 0.06 nl, about 0.07 nl, about 0.08 nl, about 0.09 nl, about 0.1 nl, about 0.2 nl, about 0.3 nl, about 0.4 nl, about 0.5 nl, about 0.6 nl, about 0.7 nl, about 0.8 nl, about 0.9 nl, about 1 nl, about 1.5 nl, about 2 nl, about 2.5 nl, about 3 nl, about 3.5 nl, about 4 nl, about 4.5 nl, about 5 nl, about 5.5 nl, about 6 nl, about 6.5 nl, about 7 nl, about 7.5 nl, about 8 nl, about 8.5 nl, about 9 nl, about 9.5 nl, about 10 nl, about 11 nl, about 12 nl, about 13 nl, about 14 nl, about 15 nl, about 16 nl, about 17 nl, about 18 nl, about 19 nl, about 20 nl, about 25 nl, about 30 nl, about 35 nl, about 40 nl, about 45 nl, or about 50 nl.

Systems for performing the above methods can include those described in WO 2014/043388.

A digital readout assay, e.g., digital analysis, can be used to count the number of template molecules generating a primer extension by partitioning the primer extension reaction molecules and identifying the partitions containing the signal generated by the primer extension. Generally, the process of digital analysis involves determining for each partition of a sample whether the partition is positive or negative for the presence of the target sequence, template molecule, or primer extension product to be detected. For quantifying the amount of target molecule in a sample (e.g., quantifying the concentration or number of copies of a target molecule in a sample), the partitions are examined for the presence of signal associated with primer extension in each partition

A detector that is capable of detecting a signal or multiple signals may be used to analyze each partition for the presence or absence of the target molecule. For example, a two-color reader (fluorescence detector) may be used. The fraction of positive-counted partitions can enable the determination of absolute concentrations for the target molecule to be measured.

Once a binary "yes-no" result has been determined for each of the partitions of the sample, the data for the partitions is analyzed using an algorithm based on Poisson statistics to quantitate the amount of target molecule in the sample. Statistical methods for quantitating the concentration or amount of a target molecule or target molecules is described, for example, in WO 2010/036352.

### Generation ofpolynucleotides having 3' stem loops or stems

Also disclosed are methods of generating a polynucleotide comprising a stem or stem loop structure at the 3' end. The stem-loop structure can be ligated (e.g., with DNA ligase) directly onto the 3' end of the DNA polynucleotide of interest. A transposable element (such as a DNA transposon or a retrotransposon) can be used to incorporate the stem-loop structure onto the 3' end of a polynucleotide.

Amplification may be used to incorporate a primer sequence into the polynucleotide of interest. Briefly, a stem-loop oligonucleotide can be used as a primer to copy a DNA template, incorporating the oligonucleotide into the 5' end of the DNA molecule and then copying back with a second oligonucleotide primer. The original strand is then removed leaving behind a single stranded DNA molecule with a stem-loop at its 3' end. This latter method is detailed more below.

The structure may be introduced by a PCR primer with a 5' region containing the reverse-complement (RC) of the non-extendable-stem-loop structure. Said another way, the primer having a stem loop at the 5' end and a 3' end that primes DNA extension by a polymerase will generate a single-stranded amplicon, whose complementary strand will be a single-stranded DNA molecule having the reverse-complement stem loop at the 3' end. A PCR primer pair may be used for amplification of a template. Both primers of the primer pair may have a 5' stem loop thereby introducing a 3' stem loop into both strands of a PCR amplicon.

FIG. 2 part A illustrates one aspect in which a stem loop primer having a 5' stem loop and a 3' sequence capable of annealing to a template DNA is annealed to a template sequence. The stem loop primer anneals to the template polynucleotide via the 3' end of the primer, which is sufficiently complementary to the template polynucleotide to allow for template-dependent extension by a DNA polymerase, thereby generating a single-stranded amplicon comprising the stem loop primer sequence linked to the complement of the template polynucleotide. A second primer can then be used to generate a complement of the single-stranded amplicon, wherein the complement will comprise at the 3' end a complement of the stem loop primer. The complement of the stem loop will nevertheless also form a stem loop, thereby resulting in an amplicon having a stem loop structure at the 3' end. The double-stranded amplicon can be rendered converted into a single stranded nucleic acid, for example by the methods described in Mitsis et al., Nucleic Acids Res (1999) vol. 27, no. 15, pp. 3057-3063; Sanchez, et al., PNAS (2004) vol. 101, no. 7, pp. 1933-1938; Chen, et al., "Asynchronous PCR", In: D. Park, ed. 2011. PCR Protocols (Methods in Molecular Biology, vol. 687). New Jersey: Humana Press Inc., pp. 231-243.

The stem loop primer(s) is composed of several sections:
At the 5' end, the stem loop can immediately begin or optionally, 1, 2, 3, 4, 5 or 6 nucleotides can be present at the 5' end that is not part of the stem loop.
The stem loop portion will comprise a sequence forming the first half of the stem, followed by a number of nucleotides forming the loop followed by a reverse complement of the first half of the stem. The first half and second half of the stem will generally be the exact same length though, one half can have one nucleotide more than the other stem half such that when the two halves anneal, one nucleotide of one half does not anneal and forms a bulge. Each half of the stem can be any size as desired, for example, between 5-15 nucleotides long, e.g., 5, 6, 7, 8, 9, 10, 11, 12, 13, 14, or 15 nucleotides. The loop will have at least one nucleotide, and in some cases, between 2-6, e.g., 2, 3, 4, 5, or 6 nucleotides. The stem can have the additional criteria described above. Alternatively, more complicates secondary structures can be formed so long as the structure impairs availability of the 3' end to reduce spurious priming.
Finally, at the 3' end of the stem loop primer, there will be a sequence that is sufficiently complementary to a target sequence to allow for annealing of the primer to the template under the conditions used. For example, the template-annealing sequence of the primer may be at least 6 nucleotides long and may be between 10-30 nucleotides, e.g., 10, 11, 12, 13, 14, 15, 16, 17, 18, 19, 20, 21, 22, 23, 24, 25, 26, 27, 28, 29, or 30 nucleotides. The entire template annealing region may be fully complementary to the template sequence. In other cases, there is for example 1, 2, 3, or more nucleotides in the annealing portion that are not complementary to the template.

Conventional techniques in molecular biology and recombinant DNA are well known and are explained in, for example, Current Protocols in Molecular Biology, 1997-2007 (F. M. Ausubel ed.), Wiley Interscience; Sambrook et al., 2001, Molecular Cloning: A Laboratory Manual, Third Edition, Cold Spring Harbor Laboratory Press, Cold Spring Harbor, N.Y.; Berger and Kimmel, Guide to Molecular Cloning Techniques Methods in Enzymology volume 152 Academic Press, Inc., San Diego, Calif. (Berger), DNA Cloning: A Practical Approach, Volumes I and II, 1985 (D. N. Glover ed.); Oligonucleotide Synthesis, 1984 (M. L. Gait ed.); Nucleic Acid Hybridization, 1985, (Hames and Higgins); Transcription and Translation, 1984 (Hames and Higgins eds.); Animal Cell Culture, 1986 (R. I. Freshney ed.); Immobilized Cells and Enzymes, 1986 (IRL Press); Perbal, 1984, A Practical Guide to Molecular Cloning*;* the series, Methods in Enzymology (Academic Press, Inc.); Gene Transfer Vectors for Mammalian Cells, 1987 (J. H. Miller and M. P. Calos eds., Cold Spring Harbor Laboratory); Methods in Enzymology Vol. 154 and Vol. 155 (Wu and Grossman, and Wu, eds., respectively).

In addition to methods of using the stem loop primer to generate an amplicon with at least one 3' end comprising a step loop, reaction mixtures for performing the methods are also provided. Reaction mixtures can contain all ingredients required for generating the amplicon with one or both 3' end of the double-stranded amplicon having a stem loop, or one or more ingredients required for completion of the reaction can be omitted. For example, all ingredients required for the method may be included in the reaction mixture except the template DNA sample. Thus, the reaction can be prepackaged such that an end user can add a nucleic acid sample and then submit the reaction mixture to conditions to allow for primer extension. The polymerase may also be omitted and can be added separately by the user when the reaction method is performed.

Also disclosed are kits for use in the methods of the invention. Typically, the kit is compartmentalized for ease of use and contains containers providing components for performing the present methods.

Also disclosed are double-stranded DNA amplicons comprising on one or both 3' ends a stem loop as described herein. The amplicons can be isolated from other amplification reaction materials (e.g., via gel electrophoresis or other methods) or can be part of a reaction mixture as described above.

### EXAMPLES

### Example 1

Human genomic DNA was used as template for 23 PCR reactions. All PCR reactions contained a fluorescently labeled forward primer and one of the following reverse primers: A. a 5'-phosphate standard reverse primer, or B. a 5'-phosphate stem-loop added to the 5' end of the reverse primer as described in Figure 1. After PCR amplification, the two amplicons were purified using standard methods. The fluorescently labeled, double-stranded amplicon was then converted to fluorescently-labeled single stranded DNA by the addition of lambda exonuclease which removed the 5'-phosphate labeled strand. Next, a quencher-labeled antisense oligo was added. The quencher-oligo was designed to hybridize to the template's fluorescent 5'-end and quench the fluorescent signal. This mixture of fluorescent single-stranded DNA and antisense-quencher was then either mixed with BST polymerase and reaction buffer, or with the equivalent no-polymerase control. The DNA's 3' priming of BST polymerase extension results in strand displacement of the antisense quencher-oligo and a high fluorescence signal. In contrast, if priming is inhibited, the (quenched) fluorescent signal remains low. The fluorescence in samples amplified using standard reverse primers was low (less than 20% higher than no-BST control) in 1 out of 6 samples tested. In contrast, the fluorescence of samples amplified using stem-loop primers was low in 13 out of 17 samples tested. This represents an improvement from 76.5% failure using standard primers to only 17% failure using stem-loop primers.

### Example 2

In one example of the method of the present invention, a PCR reaction has been performed using standard PCR reaction on KRAS gene, exon 2. The sequences of the reverse and forward primers used are respectively /5Phos/AAT TTA TAT TAA TTT ATT TAT TAT AAG GCC TGC TGA AAA TGA CTG AA (SEQ ID NO:1) and /56FAM/AGA TGC AGC AAT AAC ATG TGA ATG GTC CTG CAC CAG TAA TAT GCA TAT (SEQ ID NO:2). /5Phos/ and /56FAM/ correspond to 5'end phosphate and FAM modification respectively. After PCR amplification, reagents in excess are washed away using Qiagen purification kit or Agencourt AMPure XP beads. The DNA is mixed with Lambda exonuclease, buffer, dNTPs and the oligonucleotides Q, with sequence CACTGCCCACATGTTATTGCTGCATC/3IABkFQ/ (SEQ ID NO:3). /3IABkFQ/ modification corresponds to the 3'end addition of a black hole quencher. Lambda exonuclease is used to turn double stranded DNA into single stranded DNA, Q is designed to be complementary to the 5'end of the amplicon and quench the fluorescence of the FAM. The mix is split in to two tubes and BST polymerase is added to one the tubes. After incubation for 30 minutes at 37 degree Celsius, the fluorescence of the solutions is measured and compared. In absence of an antisense oligonucleotide, the tube with BST has a higher fluorescence of than the one without it. This indicates that the quencher oligonucleotide does not or partially quench the amplicons when BST is added; this artifact can be explained by the looping back of the template's 3'end and further extension by BST, which displaces Q from the template. In presence of an antisense oligonucleotide complementary to the 3' end of the target, with the sequence TCT ATA TAT TAA TTT ATT TA/3Phos/ (SEQ ID NO:4), the fluorescence of both tubes is identical; the antisense oligonucleotide competes with the self priming of the template, preventing the 3' end of the DNA template from being extended.

## Claims

1. A method of performing a primer extension with reduced spurious priming by a primer extension product, the method comprising
providing a reaction mixture comprising:
a single-stranded polynucleotide template comprising a 5' and a 3' end, said polynucleotide template comprising, within 6 nucleotides of the 3' end, a double stranded stem of between 5-25 nucleotides, wherein:
the double stranded stem comprises a 3' terminus and linked to the 3' terminus of the stem is 1-5 nucleotides that do not form part of the stem and that are at the 3' end of the polynucleotide template, or
a first strand of the double stranded stem is formed by a nucleotide sequence in the polynucleotide template and a second strand of the stem is a separate antisense oligonucleotide complementary to the nucleotide sequence and the antisense oligonucleotide does not comprise a 3' OH moiety or is otherwise blocked from being extended by a polymerase and wherein the antisense oligonucleotide is complementary up to the 3' end of the polynucleotide template;
a primer that anneals to a target sequence, if present, in the polynucleotide template; and
a polymerase;
exposing the reaction mixture to conditions such that the primer anneals to the target sequence, if present and the polymerase extends the primer in a template-dependent manner if the primer anneals; and
detecting the presence or amount of primer extension product.

2. The method of claim 1, wherein the double stranded stem is part of a stem loop such that both strands of the double stranded stem are linked by a loop.

3. The method of claim 1 or 2, wherein the polynucleotide template comprises one member of a quencher/fluorescent label pair and an inhibitor oligonucleotide is annealed to the polynucleotide template downstream of the primer, wherein the inhibitor oligonucleotide comprises a second member of the quencher/fluorescent label pair such that signal from the label is quenched when the inhibitor oligonucleotide is annealed to the polynucleotide template.

4. The method of claim 3, wherein the exposing comprises extending the primer with the polymerase, thereby displacing the inhibitor oligonucleotide and unquenching the quencher/fluorescent label pair to generate a fluorescent signal; and detecting the fluorescent signal and correlating the amount of signal to the presence or quantity of the target sequence.

5. The method of claim 1 or 2, wherein the polymerase is a DNA polymerase or an RNA polymerase.

6. The method of claim 5, wherein nucleotide triphosphates in the reaction mixture are incorporated into the primer extension product and at least some of said nucleotide triphosphates comprise a label.

7. The method of claim 6, wherein nucleotide triphosphates comprise a fluorescent label and quencher pair such that the fluorescent label is quenched by the quencher when the nucleotide triphosphates are intact and the fluorescent label is unquenched when the nucleotide is incorporated into the primer extension product.

8. A single-stranded polynucleotide comprising a 5' and a 3' end, said polynucleotide comprising within 6 nucleotides of the 3' end a double stranded stem of between 5-25 nucleotides, wherein:
the double stranded stem comprises a 3' terminus and linked to the 3' terminus of the stem is 1-5 nucleotides that do not form part of the stem and that are at the 3' end of the polynucleotide template, or
a first strand of the double stranded stem is formed by a nucleotide sequence in the polynucleotide template and a second strand of the stem is a separate antisense oligonucleotide complementary to the nucleotide sequence and the antisense oligonucleotide does not comprise a 3' OH moiety or is otherwise blocked from being extended by a polymerase and wherein antisense oligonucleotide is complementary up to the 3' end of the polynucleotide template.

9. A reaction mixture comprising the single-stranded polynucleotide of claim 8.

10. The reaction mixture of claim 9, wherein the polynucleotide comprises one member of a quencher/fluorescent label pair and the reaction mixture further comprises an inhibitor oligonucleotide, wherein the inhibitor oligonucleotide comprises a second member of the quencher/fluorescent label pair such that signal from the label is quenched when the inhibitor oligonucleotide is annealed to the polynucleotide.

## Patentansprüche

1. Verfahren zum Durchführen einer Primerverlängerung mit reduziertem störendem Priming durch ein Primerverlängerungsprodukt, wobei das Verfahren umfasst:
Bereitstellen eines Reaktionsgemischs, umfassend:
eine einzelsträngige Polynucleotidmatrize, die ein 5'- und ein 3'-Ende umfasst, wobei die Polynucleotidmatrize innerhalb von 6 Nucleotiden des 3'-Endes einen doppelsträngigen Stamm mit 5-25-Nucleotiden umfasst, wobei:
der doppelsträngige Stamm einen 3'-Terminus umfasst und mit dem 3'-Terminus des Stamms 1-5 Nucleotide verknüpft sind, die keinen Bestandteil des Stammes bilden und die sich am 3'-Ende der Polynucleotidmatrize befinden; oder
ein erster Strang des doppelsträngigen Stammes von einer Nucleotidsequenz in der Polynucleotidmatrize gebildet wird und ein zweiter Strang des Stammes ein separates Antisense-Oligonucleotid ist, das zu der Nucleotidsequenz komplementär ist, und das Antisense-Oligonucleotid keine 3'-OH-Gruppe umfasst oder in anderer Weise gegenüber einer Verlängerung durch eine Polymerase blockiert ist und wobei das Antisense-Oligonucleotid bis zum 3'-Ende der Polynucleotidmatrize komplementär ist;
einen Primer, der an eine Zielsequenz in der Polynucleotidmatrize assoziiert, wenn eine vorhanden ist; und
eine Polymerase;
Einwirkenlassen von Bedingungen auf das Reaktionsgemisch, bei denen der Primer an die Zielsequenz assoziiert, wenn eine vorhanden ist, und die Polymerase den Primer matrizenabhängig verlängert, wenn der Primer assoziiert; und
Nachweisen der Anwesenheit oder der Menge des Primerverlängerungsprodukts.

2. Verfahren gemäß Anspruch 1, wobei der doppelsträngige Stamm Teil einer Haarnadelstruktur ist, so dass beide Stränge des doppelsträngigen Stammes durch eine Schleife miteinander verknüpft sind.

3. Verfahren gemäß Anspruch 1 oder 2, wobei die Polynucleotidmatrize einen Partner eines Quencher/Fluoreszenzmarker-Paars umfasst und ein Inhibitoroligonucleotid stromabwärts des Primers an die Polynucleotidmatrize assoziiert ist, wobei das Inhibitoroligonucleotid einen zweiten Partner des Quencher/Fluoreszenzmarker-Paars umfasst, so dass ein von dem Marker ausgehendes Signal abgelöscht wird, wenn das Inhibitoroligonucleotid an die Polynucleotidmatrize assoziiert ist.

4. Verfahren gemäß Anspruch 3, wobei das Einwirkenlassen eine Verlängerung des Primers durch die Polymerase umfasst, wodurch das Inhibitoroligonucleotid verdrängt wird und das Quencher/Fluoreszenzmarker-Paar entlöscht wird, so dass ein Fluoreszenzsignal entsteht; und
Nachweisen des Fluoreszenzsignals und Korrelieren der Intensität des Signals mit der Anwesenheit oder Menge der Zielsequenz.

5. Verfahren gemäß Anspruch 1 oder 2, wobei die Polymerase eine DNA-Polymerase oder eine RNA-Polymerase ist.

6. Verfahren gemäß Anspruch 5, wobei Nucleotidtriphosphate in dem Reaktionsgemisch in das Primerverlängerungsprodukt eingebaut werden und wenigstens einige der Nucleotidtriphosphate einen Marker umfassen.

7. Verfahren gemäß Anspruch 6, wobei Nucleotidtriphosphate ein Fluoreszenzmarker/Quencher-Paar umfassen, so dass der Fluoreszenzmarker durch den Quencher abgelöscht wird, wenn die Nucleotidtriphosphate intakt sind, und der Fluoreszenzmarker entlöscht wird, wenn das Nucleotid in das Primerverlängerungsprodukt eingebaut wird.

8. Einzelsträngiges Polynucleotid, das ein 5'- und ein 3'-Ende umfasst, wobei das Polynucleotid innerhalb von 6 Nucleotiden des 3'-Endes einen doppelsträngigen Stamm mit 5-25-Nucleotiden umfasst, wobei:
der doppelsträngige Stamm einen 3'-Terminus umfasst und mit dem 3'-Terminus des Stamms 1-5 Nucleotide verknüpft sind, die keinen Bestandteil des Stammes bilden und die sich am 3'-Ende der Polynucleotidmatrize befinden; oder
ein erster Strang des doppelsträngigen Stammes von einer Nucleotidsequenz in der Polynucleotidmatrize gebildet wird und ein zweiter Strang des Stammes ein separates Antisense-Oligonucleotid ist, das zu der Nucleotidsequenz komplementär ist, und das Antisense-Oligonucleotid keine 3'-OH-Gruppe umfasst oder in anderer Weise gegenüber einer Verlängerung durch eine Polymerase blockiert ist und wobei das Antisense-Oligonucleotid bis zum 3'-Ende der Polynucleotidmatrize komplementär ist.

9. Reaktionsgemisch, das das einzelsträngige Polynucleotid gemäß Anspruch 8 umfasst.

10. Reaktionsgemisch gemäß Anspruch 9, wobei das Polynucleotid einen Partner eines Quencher/Fluoreszenzmarker-Paars umfasst und das Reaktionsgemisch weiterhin ein Inhibitoroligonucleotid umfasst, wobei das Inhibitoroligonucleotid einen zweiten Partner des Quencher/Fluoreszenzmarker-Paars umfasst, so dass ein von dem Marker ausgehendes Signal abgelöscht wird, wenn das Inhibitoroligonucleotid an das Polynucleotid assoziiert ist.

## Revendications

1. Procédé de réalisation d'une extension d'amorce avec un amorçage parasite réduit par un produit d'extension d'amorce, le procédé comprenant
la fourniture d'un mélange réactionnel comprenant :
une matrice polynucléotidique simple brin comprenant une extrémité 5' et une extrémité 3', ladite matrice polynucléotidique comprenant, au sein de 6 nucléotides de l'extrémité 3', une tige double brin comptant entre 5 et 25 nucléotides, dans lequel :
la tige double brin comprend une terminaison 3' et, relié à la terminaison 3' de la tige, 1 à 5 nucléotides qui ne font pas partie de la tige et qui sont au niveau de l'extrémité 3' de la matrice polynucléotidique, ou
un premier brin de la tige double brin est formée par une séquence nucléotidique dans la matrice polynucléotidique et un second brin de la tige est un oligonucléotide anti-sens séparé complémentaire à la séquence nucléotidique et l'oligonucléotide anti-sens ne comprend pas de fragment OH en 3' ou est autrement empêché d'être étendu par une polymérase et dans lequel l'oligonucléotide anti-sens est complémentaire jusqu'à l'extrémité 3' de la matrice polynucléotidique ;
une amorce qui se fixe par annelage à une séquence cible, le cas échéant, dans la matrice polynucléotidique ; et
une polymérase ;
l'exposition du mélange réactionnel à des conditions telles que l'amorce se fixe par annelage à la séquence cible, le cas échéant, et la polymérase étend l'amorce d'une manière dépendante de la matrice si l'amorce se fixe par annelage ; et
la détection de la présence ou de la quantité d'un produit d'extension d'amorce.

2. Procédé selon la revendication 1, dans lequel la tige double brin fait partie d'une tige-boucle de telle sorte que les deux brins de la tige double brin sont reliés par une boucle.

3. Procédé selon la revendication 1 ou 2, dans lequel la matrice polynucléotidique comprend un élément d'une paire extincteur/marqueur fluorescent et un oligonucléotide inhibiteur est fixé par annelage à la matrice polynucléotidique en aval de l'amorce, dans lequel l'oligonucléotide inhibiteur comprend un second élément de la paire extincteur/marqueur fluorescent de telle sorte qu'un signal en provenance du marqueur est éteint lorsque l'oligonucléotide inhibiteur est fixé par annelage à la matrice polynucléotidique.

4. Procédé selon la revendication 3, dans lequel l'exposition comprend l'extension de l'amorce avec la polymérase, déplaçant ainsi l'oligonucléotide inhibiteur et éteignant la paire extincteur/marqueur fluorescent pour générer un signal fluorescent ; et
la détection du signal fluorescent et la corrélation de la quantité de signal à la présence ou à la quantité de la séquence cible.

5. Procédé selon la revendication 1 ou 2, dans lequel la polymérase est une ADN polymérase ou une ARN polymérase.

6. Procédé selon la revendication 5, dans lequel des triphosphates nucléotidiques dans le mélange réactionnel sont incorporés dans le produit d'extension d'amorce et au moins une partie desdits triphosphate nucléotidiques comprennent un marqueur.

7. Procédé selon la revendication 6, dans lequel des triphosphates nucléotidiques comprennent une paire de marqueur fluorescent et d'extincteur de telle sorte que le marqueur fluorescent est éteint par l'extincteur lorsque les triphosphates nucléotidiques sont intacts et le marqueur fluorescent n'est pas éteint lorsque le nucléotide est incorporé dans le produit d'extension d'amorce.

8. Polynucléotide simple brin comprenant une extrémité 5' et 3', ledit polynucléotide comprenant, au sein de 6 nucléotides de l'extrémité 3', une tige double brin comptant entre 5 et 25 nucléotides, dans lequel :
la tige double brin comprend une terminaison 3' et, relié à la terminaison 3' de la tige, 1 à 5 nucléotides qui ne font pas partie de la tige et qui sont au niveau de l'extrémité 3' de la matrice polynucléotidique, ou
un premier brin de la tige double brin est formé par une séquence nucléotidique dans la matrice polynucléotidique et un second brin de la tige est un oligonucléotide anti-sens séparé complémentaire à la séquence nucléotidique et l'oligonucléotide anti-sens ne comprend pas de fragment OH en 3' ou est autrement empêché d'être étendu par une polymérase et dans lequel l'oligonucléotide anti-sens est complémentaire jusqu'à l'extrémité 3' de la matrice polynucléotidique.

9. Mélange réactionnel comprenant le polynucléotide simple brin selon la revendication 8.

10. Produit de réaction selon la revendication 9, dans lequel le polynucléotide comprend un élément d'une paire extincteur/marqueur fluorescent et le mélange réactionnel comprend en outre un oligonucléotide inhibiteur, dans lequel l'oligonucléotide inhibiteur comprend un second élément de la paire extincteur/marqueur fluorescent de telle sorte qu'un signal en provenance du marqueur est éteint lorsque l'oligonucléotide inhibiteur est fixé par annelage à la matrice polynucléotidique.
